(19) Europäisches Patentamt

European Patent Office

Office européen des brevets

(11) **EP 0 966 989 A1**

# (12) DEMANDE DE BREVET EUROPEEN

(43) Date de publication:
29.12.1999 Bulletin 1999/52

(51) Int. Cl.⁶: **A61N 5/06**

(21) Numéro de dépôt: 98401529.7

(22) Date de dépôt: 22.06.1998

(84) Etats contractants désignés:
AT BE CH CY DE DK ES FI FR GB GR IE IT LI LU MC NL PT SE
Etats d'extension désignés:
AL LT LV MK RO SI

(71) Demandeurs:
• **Chiron, Bernard**
**92190 Meudon (FR)**

• **Mondin, Jean-Daniel**
**75002 Paris (FR)**

(72) Inventeurs:
• **Chiron, Bernard**
**92190 Meudon (FR)**
• **Mondin, Jean-Daniel**
**75002 Paris (FR)**

(54) **Nouveaux equipements d'éclairage à fibres optiques pour applications de cosmétologie et photothérapie**

(57) L'invention concerne des types d'éclairage permettant de réduire des effets délétères indésirables et pendant les séances d'exposition, d'améliorer le confort des patients en diminuant les échauffements, les bruits et en créant une ambiance lumineuse calmante et reposante. Ces nouveaux équipements rendent également possibles certaines opérations de Photothérapie rélatives notamment à l'élimination des tâches sur la peau. D'un point de vue plus général, les éclairages concernés présentent une nette amélioration sur les plans ergonomiques et fonctionnels.

Les éclairages sont constitués par une source de lumière ultraviolette UVA et UVB modulable, déportée du lit-couchette, et alimentant plusieurs lignes d'éclairement constituées par des câbles à Fibres Optiques généralement plastiques équipés d'éléments optiques de focalisation. Une ligne d'éclairement spécialisée, reliée à une source secondaire pouvant délivrer une lumière visible est prévue pour des applications de Photothérapie et l'amélioration du confort des patients.

FIGURE 1

## Description

La présente invention concerne de nouveaux types d'équipements de SOLARIUM destinés aux applications de Cosmétologie et de Photothérapie délivrant des rayonnements lumineux transmis par un réseau de câbles à Fibres Optiques préférentiellement plastiques et permettant :

- d'améliorer le confort des "Patients" en diminuant considérablement la température au niveau des "couchettes-solariums" et en créant une ambiance lumineuse calmante et reposante pendant les séances d'exposition.
- de réduire notablement les effets délétéres secondaires indésirables.
- de pratiquer, grâce aux Fibres Optiques, certaines Photothérapies destinées par exemple à combattre les maladies infectieuses de la peau ou à éliminer les tâches sur la peau et notamment les "taches de vin"
- de faciliter la maintenabilité des équipements en utilisant une ou plusieurs sources de lumière, déportées des couchettes-solarium proprement dites
- d'une manière plus générale, d'améliorer la fonctionnalité des équipements, par exemple en assurant une répartition de lumière optimisée pour chaque patient et en supprimant pour ceux-çi les effets désagréables du confinement imposé par les dimensions et l'ergonomie des équipements.

Les différents équipements actuels de Solarium sont pour la plupart constitués par un bâti équipé d'une couchette pour patient et comprenant au platonnier un nombre important de tubes réflecteurs ultraviolets disposés horizontalement et de haute puissance (une quantité de 50 tubes 150 Watts étant une valeur moyenne) et pour le lit-couchette une quantité de tubes plus faible (20 à 30 tubes 100 Watts par exemple). Des lampes faciales haute pression (5 à 12 selon les équipements commercialisés actuels) d'environ 500 Watts sont également utilisées.

L'ensemble de ces tubes dissipe une puissance électrique considérable qui introduit une importante élévation de température de telle sorte que différents systèmes de ventilation sont nécessaires pour réduire la température au niveau du patient. Malgré les turbo-ventilateurs généralement utilisés, il subsiste un environnement difficile à supporter dû à une augmentation de température residuelle et au bruit supplémentaire engendré par les systèmes de refroidissement.

Par ailleurs, les équipements de solarium existants sont l'objet de violentes critiques dans la mesure où il est démontré que les radiations ultraviolettes utilisées (longueurs d'onde et intensités de radiations lumineuses correspondantes) sont génératrices, à terme, d'effets Carcinogènes, Mutagènes, Erythématogènes et Pigmentogènes.

De plus, les radiations ultraviolettes sont émises par des Tubes réflecteurs dont l'intensité lumineuse ne peut être modifiée pendant les séances d'exposition ou avant chaque séance. La durée d'exposition reste ainsi le seul paramètre variable permettant d'atteindre l'effet cosmétique récherché.

Du point de vue de la maniabilité, les équipements de solarium commercialisés, compte tenu de leur poids, voisin de 400 Kgs, ne sont pas déplaçables facilement d'une cabine à l'autre, et ne possèdent pour la plupart qu'une position d'éclairement horizontale.

Des équipements légers et mobiles pouvant occuper une position verticale qui améliorerait le confort pour les patients atteints, par exemple, de psoriasis, sont souhaités par le personnel esthéticien des instituts utilisateurs.

Les objectifs généraux de l'invention concernent la réalisation de nouveaux types d'équipements, éventuellement mixtes pour des applications à la fois de cosmétologie et de Phototherapie, grâce à l'emploi de Fibres Optiques utilisées sous forme d'un faisceau de plusieurs câbles alimenté par une source de lumière extérieure, déportée du support des extrémités éclairantes des câbles à Fibres optiques d'une distance au plus égale à la longueur de ceux-çi.

Si nécessaire, les extrémités éclairantes sont elles-mêmes équipées :

- soit unitairement de composants optiques déviateurs et focalisateurs de lumière.
- soit de composants optiques telles des lentilles de Fresnel de grande surface agissant sur la lumière émise par un groupe d'extrémités éclairantes.

Selon un objet non limitatif de l'invention, les Fibres Optiques Plastiques sont préférentiellement utilisées pour la réalisation des Faisceaux de câbles optiques. Ces Fibres Optiques Plastiques présentent notamment l'avantage d'une forte absorption dans le domaine des infrarouges (par exemple une transmission inférieure à 0,01% de la transmission totale à la longueur d'onde de 850 nm) et l'effet thermique de la lumière émise (notamment augmentation de la température dans l'environnement du patient) est donc considérablement réduit.

Cette réduction est d'autant plus nécessaire et appréciée que le spectre de longueurs d'onde utilisées est plus proche du spectre infrarouge, ce qui peut être le cas notamment dans les applications de Photothérapie.

Dans tous les cas, le déport de la source lumineuse selon l'invention, à distance du patient, élimine totalement l'élévation de température due à la source proprement dite.

Par ailleurs, les Fibres Optiques Plastiques, au contraire des fibres à base de Silice ou quartz ne sont pas fragiles. Une Fibre Optique Plastique de 1 mm de diametre, par exemple, peut subir un nombre considérable de courbures de rayon égal à 10 mm sans qu'aucune rupture ne soit constatée.

Ainsi, la souplesse d'emploi et l'excellente résistance mécanique à la flexion et à la traction des Fibres Optiques Plastiques permettent, sans connectiques associées, une séparation du Faisceau principal de Fibres Optiques en plusieurs autres Faisceaux de plus faible diamètre, ceux-çi étant eux-mêmes subdivisés en nombreux câbles optiques élémentaires diffuseurs de lumière. Il est aussi possible de modifier, très fréquemment, en fonction de l'effet cosmétique recherché la position respective de chaque embout éclairant sans aucune détérioration mécanique, tandis que la perte de transmission reste inchangée entre 0,15 et 1dB par mètre dans tout le spectre visible, incluant les rayonnements ultraviolets jusqu'à 280 nm.

L'invention sera mieux comprise à partir des Figures qui suivent.

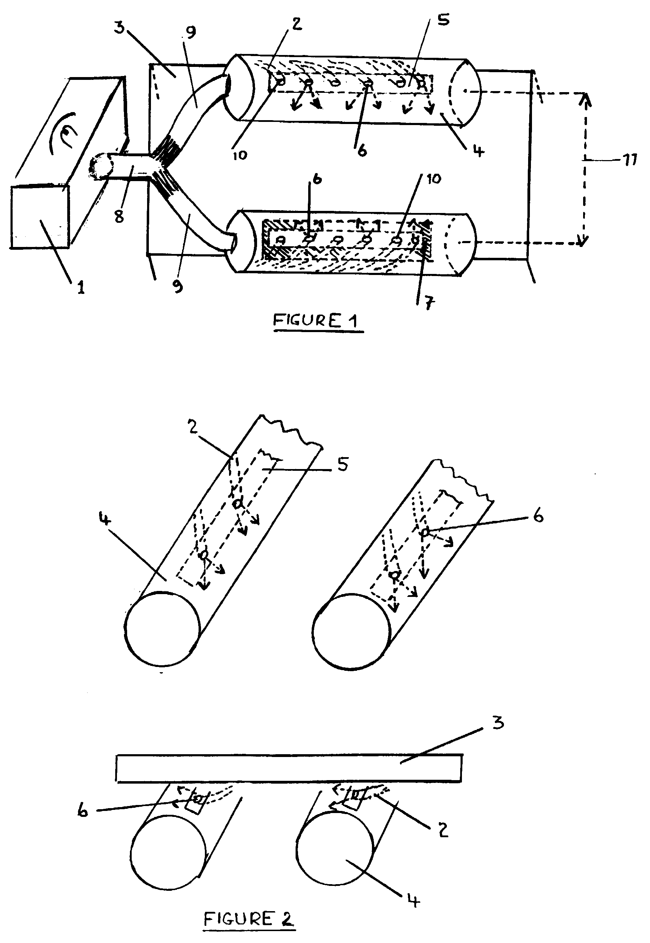

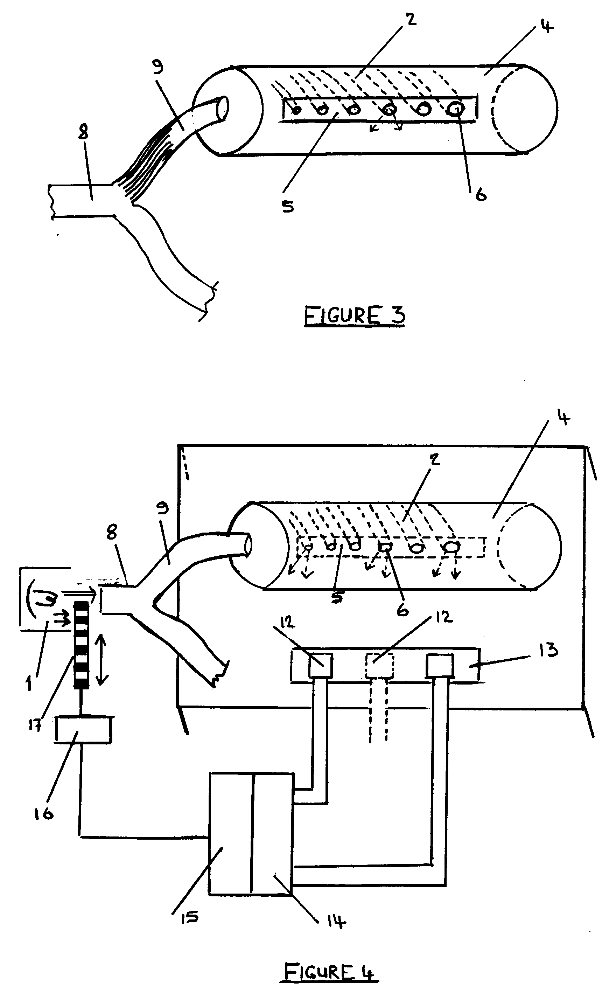

La Figure 1 montre très schématiquement suivant l'invention, un système d'éclairage par Fibres Optiques pour Solarium. Une source de lumière 1 est raccordée à un Faisceau de Fibres Optiques principal 8 séparé en 2 Faisceaux 9 eux-mêmes subdivisés en un nombre égal de câbles optiques élémentaires 2 dont les extrémités éclairantes 6 diffusent la lumière sur le lit-couchette 3 du patient. Les câbles optiques 2 sont solidaires d'un support 4 muni d'une glissière 5 dans laquelle peuvent coulisser les extrémités éclairantes 6 qui sont ensuite immobilisées par tous moyens mécaniques simples connus sur la glissière 5 avec l'écartement nécessaire entre les extrémités éclairantes 6, en fonction de l'éffet cosmétique souhaité.

Les extrémités éclairantes 6 sont généralement équipées d'embouts optiques 10, avec lentilles déplacables par rapport à la face de sortie des câbles 2. Les embouts 10 sont orientables mécaniquement et suivant un exemple non limitatif de l'invention par un système à rotule.

Les embouts 10 permettent notamment de modifier l'amplitude et la dimension de la surface d'éclairement délivrée par chaque câble 2.

Il est ainsi possible d'adapter les caractéristiques de l'éclairement en fonction du type de peau, de la pilosité, des éventuelles affections inesthétiques, voire infectieuses de la peau.

Le système d'éclairage montré sur la Figure 1 permet de satisfaire au mieux à toutes les exigences du patient relatives à la qualité du bronzage qu'il recherche.

Selon la Figure 1 qui donne un exemple non limitatif de l'invention, l'écartement 11 entre les 2 lignes d'éclairement par Câbles Optiques 2 est d'environ 100 à 120 cms et est ainsi supérieur à la largeur du lit-couchette 3, de l'ordre de 80 cms. Ceçi permet une excellente uniformité de l'éclairement sur toute la surface du corps, le patient devant cependant accepter de se retourner pour obtenir un bronzage complet.

Selon une autre version de la Figure 1, l'écartement 11 entre les lignes d'éclairement est, au contraire de la disposition précédente, inférieur à la largeur de la couchette 3. A titre d'exemple non limitatif, un écartement 11 d'environ 30 cms entre les lignes d'éclairement permet de doubler l'intensité lumineuse sur le lit-couchette 3 grâce à la coincidence des surfaces d'éclairement formées par chaque ligne des câbles 2.

Selon un autre objet de l'invention relative à la Figure 1, l'utilisation des câbles à Fibres Optiques Plastiques présentant une grande souplesse d'utilisation permet sans risques optiques et mécaniques de réaliser un équipement unique dont l'écartement entre les 2 lignes d'éclairage varie à titre non limitatif entre 30 et 120 cms par exemple. Compte tenu de l'extrême résistance mécanique des Fibres Optiques Plastiques, la variation de l'écartement 11 peut être obtenue suivant tous moyens électromécaniques connus et simplement, par exemple, par un déplacement des supports 4, l'un par rapport à l'autre.

La Figure 2 montre suivant un autre objet de l'invention un éclairage simultané de l'ensemble des parties du corps en utilisant deux systèmes d'éclairage identiques à celui montré sur la Figure 1, soit 4 lignes d'éclairement.

Une seule source de lumière peut être employée si l'éclairement délivré par les 4 lignes de câbles élémentaires 2 est suffisant pour obtenir les effets cosmétiques recherchés. Dans le cas contraire, on utilise 2 sources, chaque source alimentant 2 lignes d'éclairement.

Comme il est montré sur la Figure 2, chaque groupement de 2 lignes d'éclairement est placé dessus ou dessous le lit-couchette 3.

Le matériau du lit-couchette 3 doit être le plus transparent possible à la lumière ultraviolette et suivant un exemple non limitatif de l'invention être constitué par un matériau du type Polycarbonate (PC) ou du type Polyméthylméthacrylate (PMMA) éventuellement dopé par de l'acétate de Butyle. Le taux d'Hydroquinol nécessaire à la polymérisation du PMMA pur ou dopé devra, compte tenu du spectre de longueur d'onde utilisé être faible et par exemple inférieur à 1%.

Selon les Figures 1 et 2 relatives à l'invention, on peut améliorer, si nécessaire, l'homogénéité de l'éclairement sur le lit-couchette 3 en plaçant devant les extrémités éclairantes 6, un matériau diffusant 7 utilisé par exemple sous forme d'une plaque de faible épaisseur (quelques mm) située à proximité des extrémités éclairantes 6. Des coéfficients de diffusion de 40 à 85% peuvent être obtenus avec les matériaux plastiques diffusants, actuellement commercialisés.

Selon les Figures 1 et 2 les sources de lumière sont constituées d'une ou plusieurs lampes délivrant une lumière ultraviolette et généralement associées à un réflecteur. Le couplage optimal de la source 1 au Faisceau principal 8 est obtenue par une association de lentilles collimatrices, de filtres de bande et de filtres anticaloriques qui éliminent les infrarouges résiduels et limitent ainsi la température à l'entrée du Faisceau 8.

Selon un autre objet de l'invention montré sur la Figure 3 où, pour simplifier la Figure, seule une ligne de câbles optiques élémentaires 2 issus d'un Sous-Faisceau 9 est figurée, on utilise une ligne de câbles optiques 2 dont certains des diamètres, ou la totalité de ceux-çi sont différents. En particulier, les extrémités 6 des câbles optiques éclairant la partie supérieure du patient (face et torse) ont un diamètre plus important que ceux éclairant la partie basse du patient (jambes) et diffusent ainsi une intensité lumineuse beaucoup plus grande que celle reçue sur le reste du corps. Le rôle joué par ces câbles optiques 2, de plus grand diamètre, s'apparente ainsi à celui des lampes faciales utilisées dans les équipements de solarium traditionnels actuels.

Selon un autre objet de l'invention, le Figure 4 montre schématiquement un système de régulation automatique de l'éclairement du Patient par Câbles à Fibres Optiques. Une ou plusieurs photodiodes 12 régulièrement réparties le long d'une règlette support 13 préférentiellement solidaire du lit-couchette, mais pouvant être séparée, delivrent des informations de courant dont l'amplitude est d'autant plus élévée que la lumière détectée par les photodiodes est plus importante.

Les différents courants des Photodiodes sont additionnés dans un additionneur 14, amplifiés dans un amplificateur 15 lequel actionne un moteur 16 commandant, suivant un mouvement rectiligne de translation, un disque atténuateur passif 17 occultant une partie de la lumière émise par la source 1.

Si la lumière captée par les photodiodes diminue, la puissance du moteur 16 est plus faible et l'occultation de la lumière par le disque atténuateur 17 est réduite et inversement. Des variations de lumière supérieures à 40% peuvent être obtenues.

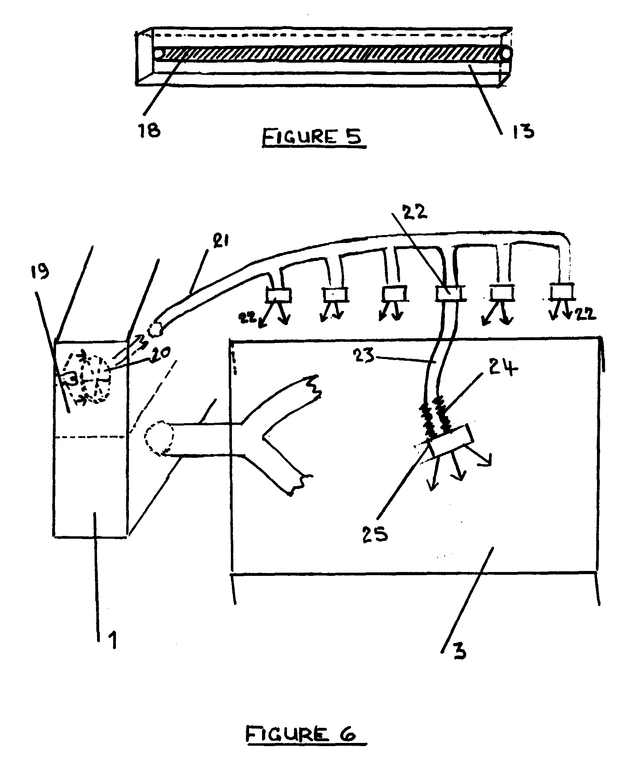

La Figure 5 montre selon l'invention, une autre version de la règlette de captation de lumière 13. Cette règlette est constituée essentiellement par un matériau fluorescent 18 de forme longiligne, la forme de la section n'étant pas prépondérante. On utilise préférentiellement, selon l'invention et à titre non limitatif une fibre fluorescente de section circulaire réalisée en matériau Polystyrène de haute transparence dopé faiblement ($10^{-4}$ par exemple) par des produits fluorescents notamment du type coumarine (rouge), K27 (Vert).

Sous l'influence d'un rayonnement de longueur d'onde $\lambda1$, le matériau fluorescent délivre à ses 2 extrémités une lumière de longueur d'onde $\lambda2$ telle que $\lambda2 > \lambda1$.

La lumière de longueur d'onde $\lambda2$ peut être détectée par une simple photodiode que l'on associe au système de régulation automatique décrit précédemment.

A titre d'exemple, une fibre fluorescente de diamètre 1 mm soumise à un éclairement de 400 lux (dans l'UV) délivre à chacune de ses extrémités une lumière rouge d'une puissance de 40 µW.

La Figure 6, montre selon un autre objet de l'invention, un système d'apport de lumière visible supplémentaire, destiné au confort du patient.

La lumière UV utilisée pour l'obtention des effets cosmétiques sur les patients n'étant pas visible, il en résulte pour certains de ceux-çi un sentiment d'inquiétude faisant rapidement place à un stress plus ou moins supportable. Pendant la séance de bronzage UV un apport de lumière visible tamisée, de faible intensité est de nature à créer un apaisement salutaire.

Le système d'apport de lumière peut être simplement constitué par une série de lampes éventuellement teintées. Cependant, selon une disposition préférentielle de l'invention on utilise comme il est montré sur la Figure 6, un générateur mixte constitué d'une part par la source 1 délivrant la lumière ultraviolette nécessaire à l'obtention des effets cosmétiques recherchés et d'autre part par une source secondaire 19 de plus faible puissance et équipée d'un disque 20 à secteurs de couleurs différentes commandé mécaniquement ou par moteur. La source 19 est raccordée à un nouveau câble 21 (ou plusieurs) à Fibres Optiques placé, à titre non limitatif, dans le plan de symétrie vertical du lit-couchette 3 et comprenant plusieurs sorties éclairantes 22 en lumière visible équipées éventuellement d'optiques de focalisation ou de translation du faisceau de lumière émis.

La source 19 délivre une lumière d'ambiance colorée dépendant de l'orientation du disque de couleurs 20 par rapport à la lampe.

Selon un autre objet de l'invention, montré également sur la Figure 6, un câble 23 spécialement destiné à des opérations de Photothérapie est raccordé à l'une des sorties éclairantes 22 du câble 21. A titre d'exemple non limitatif, ce câble est utilisé pour le traitement des tâches de vin qui touchent 0,5% de la population.

Le traitement s'effectue avant ou après la séance de bronzage. Dans ce cas, le secteur du disque coloré 20 de la source 19 sera placé sur la position de couleur rouge.

La longueur d'onde du filtre de couleur correspondant sera préférentiellement choisie entre 580 et 620 nm.

Dans cette bande de longueur d'onde, l'énergie lumineuse est préférentiellement absorbée par l'hémoglobine circulant dans le sang. Sous l'influence de cette irradiation, les vaisseaux constituant les tâches de vin se coagulent et disparaissent sans laisser de trace.

La longueur du câble 23, doit être suffisante pour permettre à l'opérateur médical d'approcher l'extrémité éclairante 25 équipée de lentilles de focalisation à proximité immédiate de la tâche de vin. Le câble 23 peut être équipé d'une enveloppe semi-rigide 24 permettant à l'extrémité éclairante 25 de garder une position fixe sans la présence de l'opérateur médical.

Les équipement de Solarium actuels sont essentiellement basés sur l'utilisation de rayonnements ultraviolets A et B qui permettent l'obtention des effets cosmétiques de bronzage recherchés. Cependant, il existe dans les milieux médicaux spécialisés et particulièrement chez les chercheurs, une réelle unanimité sur le fait que ces rayonnements produisent, à certaines longueurs d'onde une assez grande diversité de dangers.

Un nouvel objet essentiel de l'invention, concerne des équipement Solarium à Fibres Optiques émettant des rayonnements ultraviolets à des longueurs d'onde bien particularisées et dont la nocivité est très réduite tout en satisfaisant aux exigences cosmétiques.

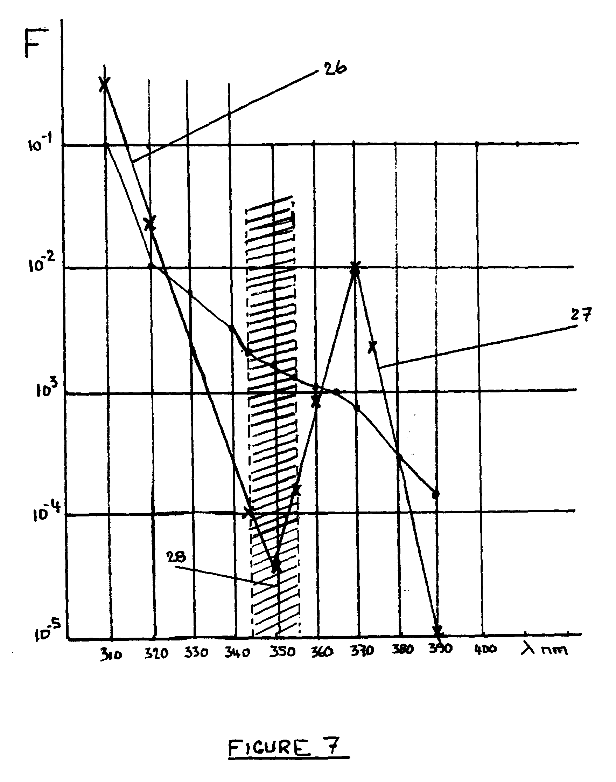

La Figure 7 justifie le choix des longueurs d'onde ultraviolettes concernées par l'invention. Les courbes 26 et 27 de cette Figure rélatives respectivement aux effets érythematogènes et carcinogènes montrent comment le facteur d'innocuité

$$F = \frac{\text{Action à chaque longeur d'onde}}{\text{Action maximale (à } \lambda = 300 \text{ nm)}}$$

défini, dans le cadre de l'invention, à partir d'informations les plus crédibles, varie avec la longueur d'onde dans le cas des 2 effets carcinogènes et érythématogènes les plus graves.

Un Facteur d'innocuité F = 1 est significatif d'effets néfastes très importants tandis que F = 0 traduit une innocuité totale.

La zône hachurée 28 de la Figure 7 montre, selon l'invention, une bande de longueurs d'onde des UVA préférentielle qui se situe entre 345 et 355 nm.

Dans cette bande de longueur d'onde, le facteur d'innocuité est environ 1000 fois inférieur au Facteur d'innocuité maximum à $\lambda = 300$ nm. Par ailleurs, les effets carcinogènes sont environ 15 fois plus faibles que les effets érythématogènes et pigmentogènes.

De manière à faciliter l'apparition des effets cosmétiques du bronzage et à en prolonger le résultat, on ajoute, selon l'invention, simultanément à l'énergie UVA, une faible dose d'énergie UVB.

Comme le montre la Figure 7, une longueur d'onde UVB voisine de 310 nm qui est par ailleurs une longueur d'onde où l'efficacité du soleil est proche de son maximum est favorable.Cependant, son facteur d'innocuite $F = 10^{-1}$ est trop élevé et l'énergie UVB à 310 nm doit être réduite de 100 fois par rapport à celle irradiée dans la bande de longueur d'onde UVA choisie.

Les caractéristiques des rayonnements UVA + UVB utilisés selon l'invention sont données à titre d'éxemple dans le tableau ci-dessous qui n'est pas limitatif: le temps d'irradiation et la puissance rayonnée étant notamment liés.

| Longueur d'onde (nm) | Puissance rayonnée (Watt/m2) | Temps d'irradiation (mn) | Energie (Joules/m2) |
|---|---|---|---|
| UVA : 345 à 355 | 0,15 | 11 | 100 |
| UVB : 310 | 0,0015 | 11 | 1 |

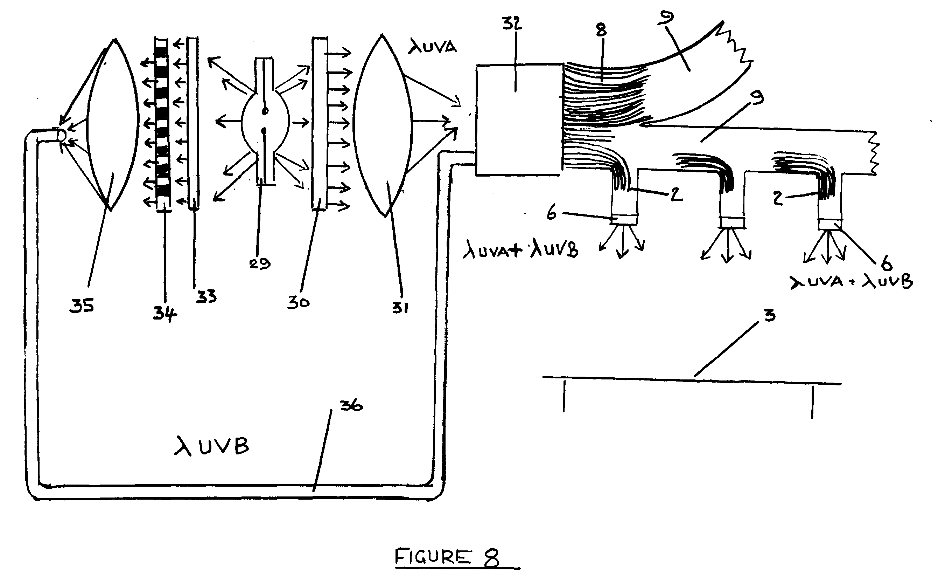

La Figure 8 montre selon l'invention un exemple très simplifié d'équipement permettant de délivrer une irradiation mixte UVA + UVB.

Une lampe UV 29 fonctionne sans réflecteur et diffuse des rayonnements de part et d'autre de la lampe.

D'un coté, un filtre de bande 30 sélectionne la bande de longueur d'onde UVA choisie et une lentille 31 concentre le rayonnement UVA dans un barreau mélangeur 32 réalisé en matériau transparent.

Du coté opposé, un filtre de bande étroite 33 sélectionne la longueur d'onde UVB choisie. Un atténuateur 34 passif, fixe, à trous, atténue le rayonnement UVB dans un rapport voisin de 100 par rapport à l'énergie UVA.

Une lentille 35 concentre ensuite le rayonnement UVB dans un câble à Fibres Optiques Plastiques 36 raccordé au barreau mélangeur 32. Le reste de l'équipement est ensuite conforme à la Figure 1. Cependant chaque extrémité éclairante délivre simultanément à la fois une radiation dans les bandes UVA et UVB (certains éléments comme les glissières-supports des éxtrémités éclairantes 6 ne sont pas figurées par souçi de simplification).

La Figure 9 montre la variation typique du rapport de transmission

$$R = \frac{\text{Puissance de sortie}}{\text{Puissance d'entrée}}$$

des Fibres Optiques Plastiques en fonction de la longueur d'onde dans le domaine des Ultraviolets pour une longueur de 5 mètres. On remarque que dans la gamme des UVA, de 345 à 355 nm, le rapport de transmission est de l'ordre de 70% mais est réduit à moins de 40% dans la gamme des UVB à partir de 300 nm. Il existe donc un "effet de dispersion" avec les Fibres Optiques Plastiques qui peut, selon un objet de l'invention, être favorablement exploité dans la mesure où la perte de transmission de ces Fibres Optiques Plastiques est d'autant plus grande que le Facteur d'innocuité F (ou les effets biologiques néfastes) croît quand la longueur d'onde des ultraviolets diminue.

Certains experts ont démontré qu'une dose d'ultraviolet semble plus carcinogène quand elle est délivrée à basse énergie pendant un temps prolongé. Corrélativement, d'autres scientifiques ont également remarqué que les liaisons dimériques créées par les ultraviolets pouvaient être réduites d'un "Ordre de Grandeur" quand ils utilisaient dans leurs recherches des Lasers ultraviolets délivrant pendant un temps très court une énergie de haute intensité.

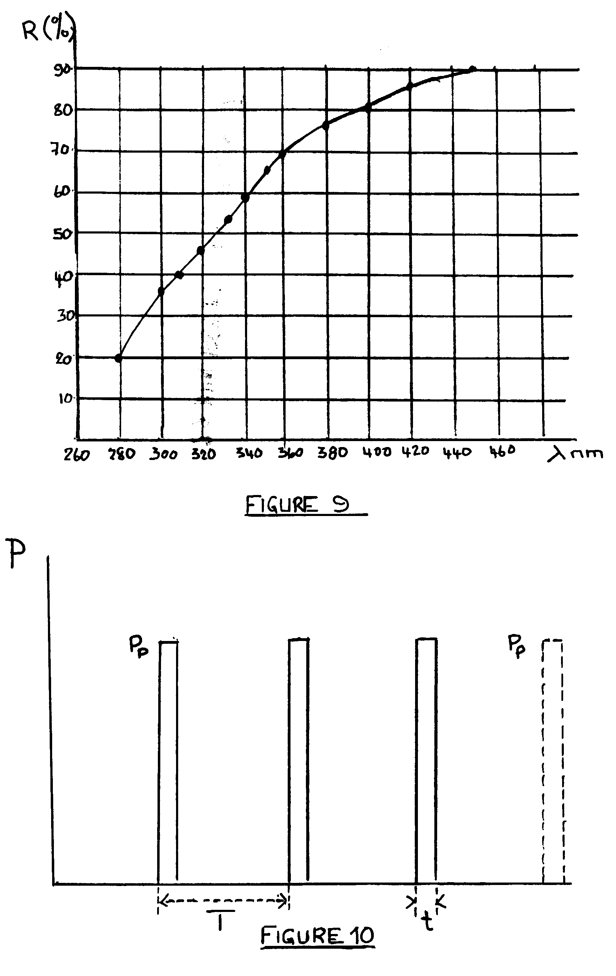

Selon un autre objet de l'invention, la source de lumière ultraviolette délivre une intensité d'irradiation comme il est montré sur la Figure 10, et à titre non limitatif, sous formes d'impulsions identiques, répétées suivant un cycle périodique.

Dans ce cas, le "temps de stimulation" des effets biologiques, que l'on peut appeler "trainage" peut être plus long que la durée t de chaque impulsion de lumière diffusée de Puissance Pp. Ainsi, un décalage de temps est introduit entre la durée de chaque impulsion et le seuil de déclenchement des effets biologiques, augmenté d'un "temps de pause " T fixé par la fréquence de répétition de chaque impulsion.

Si l'on considère, suivant la Figure 10, une irradiation de lumière pendant un temps total T, l'Energie irradiée Ep en régime pulsé est

$$Ep = \frac{Pp \; x \; t \; x \; T}{T}$$

A titre d'exemple pratique et non limitatif de réalisation d'un équipement du type Solarium à Fibres Optiques Plastiques selon l'invention, fonctionnant en régime continu, on utilise préférentiellement une source constituée par une lampe à vapeur de Mercure et à arc court, du type OSRAM HBO R 103W/45 équipée d'un réflecteur. Cette technologie de lampe est utilisée à titre non limitatif, mais présente l'avantage de délivrer une puissance importante dans le spectre ultraviolet et plus particulièrement aux longueurs d'ondes préconisées dans le cadre de l'invention.

L'irradiation lumineuse de la lampe est dirigée dans une seule direction et est focalisée dans un plan où est placée l'entrée du Faisceau de Fibres Optiques Plastiques.

Les lampes à vapeur de mercure peuvent délivrer, en fonction de la puissance normale d'alimentation et dans l'ensemble de leur spectre d'émission, des Flux lumineux supérieurs à 45000 lumens, ceux-çi restant supérieurs à 10000 lumens dans le spectre des ultraviolets A et B.

Dans notre exemple pratique, une longueur de câbles moyenne de 3,50 mètres a été choisie entre la sortie de la source et les extrémités éclairantes des câbles optiques élémentaires qui sont au nombre de 12, répartis suivant 2 lignes d'éclairement parallélles.

Le Faisceau principal de Fibres Optiques Plastiques utilise au total 420 fibres, d'un diamètre unitaire de 1 mm, compactées à l'extrémité du Faisceau couplé à la source, dans un embout de diamètre intérieur de 23 mm.

Compte tenu de la perte de transmission dans les câbles optiques, on recueille à l'extrémité de chaque sortie éclairante une intensité lumineuse de 380 lumens.

Chaque câble élémentaire comprend un nombre de Fibres Optiques Plastiques identique et égal à 35 et présente un diamètre de 6,5 mm. Les dimensions du lit-couchette (180 x 96 cms) prises en compte conduisent à une surface totale d'éclairement nécessaire de 1,728 m2.

Il en résulte que la puissance totale lumineuse exprimée en Watt/m2 est d'environ 130 Watts/m2 à proximité des extrémités éclairantes des câbles élémentaires. Si on place le lit-couchette du solarium à environ 100 cms des extrémités éclairantes, la puissance lumineuse de la lumière irradiant le patient décroit et est réduite à 0,32 Watts/m2.

Compte tenu des équipements annexes comme le système de régulation automatique de niveau de lumière, l'utilisation et l'emploi d'une lampe sans réflecteur, d'un barreau mélangeur des longueurs d'onde UVA et UVB, la puissance lumineuse irradiant le patient ne dépasse pas 0,15 Watts/m2 soit pour une énergie souhaitée de 100 joules, une durée d'exposition de l'ordre de 11 minutes.

**Revendications**

1. Système mixte d'éclairement pour applications de cosmétologie et Photothérapie particulièrement de bronzage artificiel et de traitement de maladies de la peau, notamment l'élimination des tâches telles les tâches de vin, caractérisé en ce qu'il comprend des câbles élémentaires à Fibres Optiques préférentiellement Plastiques reunis à une de leurs extrémités sous forme d'un Faisceau (8), lui-même relié à une ou plusieurs sources lumineuses (1) déportées des autres extrémités éclairantes des câbles à Fibres Optiques (2) délivrant les irradiations nécessaires et en ce qu'il comprend un ensemble de dispositifs optoélectroniques et mécaniques constituant un système multifonctions permettant d'effectuer des opérations de régulation, de modulation de la puissance irradiée et d'un point de vue plus général de modifier les caractéristiques d'éclairement des patients.

2. Système mixte d'éclairement selon la revendication 1, caractérisé en ce qu'il est constitué par plusieurs lignes d'éclairement, généralement 2 ou 4, équipées chacune d'une glissière (5), dans laquelle peuvent être déplacées les extrémités éclairantes (6) des câbles élémentaires à Fibres Optiques (2), les lignes d'éclairement étant par ailleurs mobiles les unes par rapport aux autres.

3. Système mixte d'éclairement selon les Revendications 1 et 2, caractérisé en ce que la section utile d'éclairement d'une partie ou de la totalité de chacun des câbles élémentaires à Fibres Optiques (2) est différente, ceux-çi délivrant dans ce cas une irradiation d'intensité différente.

4. Système mixte d'éclairement selon l'une quelconque des revendications 1 à 3, caractérisé en ce que l'ensemble des dispositifs optoélectroniques et mécaniques comprend un système de régulation doté de capteurs de lumière du type Photodiodes (12) placés dans le champ lumineux et associés à des dispositifs électroniques de détection et d'amplification (14, 15, 16, 17) qui permettent de conserver un niveau de lumière irradié constant sur le patient.

5. Système mixte d'éclairement selon la revendication 4 caractérisé en ce que les capteurs de lumière sont des matériaux fluorescents notamment de forme filaire tels des Fibres Optiques Fluorescentes (18).

6. Système mixte d'éclairement selon l'une quelconque des revendications 1 à 5 caractérisé en ce que l'énergie produite par les sources lumineuses (1) pour l'irradiation du patient est constituée simultanément d'une énergie UVA dans une bande de longueur d'onde comprise entre 345 et 355 nm et d'une énergie UVB à une longueur d'onde voisine de 310 nm, l'énergie UVB étant environ 100 fois inférieure à celle émise dans la bande UVA.

7. Système mixte d'éclairement selon l'une quelconque des revendications 1 à 6, caractérisé en ce qu'il comporte un éclairage supplémentaire destiné au confort du patient et constitué par une nouvelle ligne d'éclairage de câbles (21) à Fibres Optiques alimentée par une source secondaire (19) de lumière visible dont il est possible de modifier la couleur et l'intensité (20).

8. Système mixte d'éclairement selon la revendication 7, caractérisé en ce qu'il comprend un câble supplémentaire (23) raccordé à l'une des extrémités éclairantes (22) de la ligne d'éclairage "de confort" (21), la lumière, généralement visible, nécessaire au traitement, étant délivrée directement par la source secondaire de lumière (19) ou par l'intermédiaire d'un filtre de couleur (20) placé en extrémité du câble de Photothérapie.

9. Système mixte d'éclairement, selon les revendications 1 à 8, caractérisé en ce que les sources lumineuses fournissent une intensité lumineuse sous forme d'impulsions répétées de très faible durée suivant un cycle périodique et de très forte puissance par rapport à celle délivrée d'une manière continue.

FIGURE 1

FIGURE 2

FIGURE 3

FIGURE 4

18                    FIGURE 5                    13

FIGURE 6

FIGURE 7

FIGURE 8

FIGURE 9

FIGURE 10

**Office européen des brevets**

**RAPPORT DE RECHERCHE EUROPEENNE**

Numéro de la demande

EP 98 40 1529

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | DE 195 02 980 A (IMAB-STIFTUNG) 1 août 1996 * abrégé * * page 2, colonne 26 - colonne 38 * * colonne 3, ligne 34 - ligne 41; revendication 3 * | 1 | A61N5/06 |
| A | EP 0 612 539 A (KÖNIG) 31 août 1994 * abrégé * | 1 | |
| A | US 5 300 097 A (LERNER) 5 avril 1994 * abrégé * | 1 | |
| A | US 5 344 433 A (TALMORE) 6 septembre 1994 * colonne 2, ligne 56 - colonne 3, ligne 7 * | 1 | |

| | DOMAINES TECHNIQUES RECHERCHES (Int.Cl.6) |
|---|---|
| | A61N |

Le présent rapport a été établi pour toutes les revendications

| Lieu de la recherche | Date d'achèvement de la recherche | Examinateur |
|---|---|---|
| LA HAYE | 27 novembre 1998 | Taccoen, J-F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
 
& : membre de la même famille, document correspondant

EPO FORM 1503 03.82 (P04C02)